# EUROPÄISCHE PATENTSCHRIFT

(11) **EP 3 235 805 B1**
(45) Veröffentlichungstag und Bekanntmachung des Hinweises auf die Patenterteilung: **04.07.2018**
(21) Anmeldenummer: 16165898.4
(22) Anmeldetag: 19.04.2016
(51) Int. Cl.: C07C 209/84, C07C 211/36, B01D 3/10, B01D 3/14, B01D 5/00

(54) **KOPPLUNG DER DESTILLATIVEN AUFREINIGUNG MIT EINEM PARTIALKONDENSATOR ZUR VORREINIGUNG VON ISOPHORONDIAMIN**
COUPLING THE DISTILLATIVE PURIFICATION WITH A PARTIAL CAPACITOR FOR PRE-PURIFICATION OF ISOPHORONDIAMINE
COMBINAISON DU NETTOYAGE PAR DISTILLATION COMPRENANT UN CONDENSEUR PARTIEL DESTINE AU PRE-NETTOYAGE D'ISOPHORONEDIAMINE

(43) Veröffentlichungstag der Anmeldung: 25.10.2017
(73) Patentinhaber: Evonik Degussa GmbH, 45128 Essen (DE)
(72) Erfinder: RITTSTEIGER, Anne, 59399 Olfen (DE); RÜFER, Alexander Martin, 45657 Recklinghausen (DE); STREUKENS, Guido, 42111 Wuppertal (DE); KOHLSTRUK, Stephan, 45966 Gladbeck (DE); ORSCHEL, Matthias, 48161 Münster (DE); MENDORF, Matthias, 44329 Dortmund (DE); WALSCHARTS, Jorn, 2650 Edegem (BE); HENGSTERMANN, Axel, 48308 Senden (DE); MÜLLER, Anja, 44135 Dortmund (DE)

(56) Entgegenhaltungen:
- EP-A1- 2 649 042
- CN-A- 104 292 112
- DE-A1- 10 236 675
- JP-A- S62 114 943

## Beschreibung

Die Erfindung betrifft die Feinreinigung von Isophorondiamin (IPDA) mit Hilfe eines zweistufigen Kolonnenaufbaus mit Partialkondensator.

Die Herstellung von IPDA durch aminierende Hydrierung von Isophoronnitril (IPN) ist bekannt und wurde bereits mehrfach beschrieben.

Im einfachsten Fall (US 3,352,913) wird IPN in Gegenwart von Wasserstoff und eines Überschusses an Ammoniak an einem Cobaltkatalysator zur Reaktion gebracht. Zunächst bildet sich aus IPN und Ammoniak durch Wasserabspaltung das Isophoronnitrilimin, IPNI, das nachfolgend zum IPDA hydriert wird.

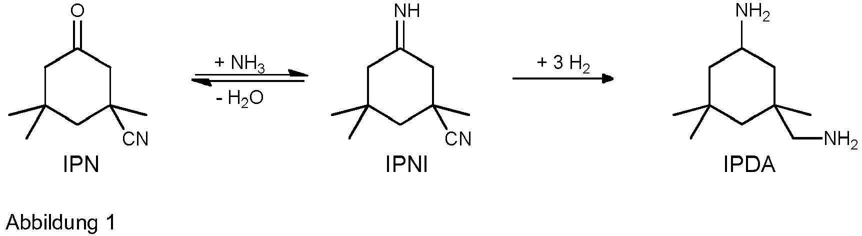

Weiterhin sind Verfahren zur Herstellung von Isophorondiamin aus CN 104230721A, EP 2649042A und WO 2012126869A bekannt.

Isophorondiamin wird gemäß der EP 2 649 042A in einer ein- oder zweistufigen Reaktion aus Isophoronnitril hergestellt. Dabei wird Isophoronnitril zunächst mit Ammoniak zu Isophoronnitrilimin iminiert. Im zweiten Schritt wird dieses zu Isophorondiamin hydriert. Die an die Reaktion angeschlossene Aufreinigung gliedert sich ebenfalls in zwei Schritte. Zunächst werden in mehreren Destillationskolonnen die Leichtsieder abgetrennt, dazu gehören Wasserstoff, Inertgase, Ammoniak und leichtsiedende Verunreinigungen (Leichtsiederabtrennung). In einem letzten Schritt wird dann das Rein-Isophorondiamin durch zwei Vakuumdestillationskolonnen gewonnen. Die erste Kolonne dient wiederum der Abtrennung von noch enthaltenen leichter siedenden Nebenprodukten. In der zweiten Kolonne wird das Isophorondiamin rein über Kopf gewonnen und so von den organischen Rückständen (Schwersieder) getrennt.

Aufgabe der vorliegenden Erfindung ist es, ein einfaches Verfahren zur Feinreinigung von Isophorondiamin zu finden mit reduziertem Energiebedarf in Form von Heiz- und Kühlleistung.

Überraschenderweise wurde gefunden, dass sich durch den Einsatz eines zusätzlichen Partialkondensators in der ersten Vakuumdestillationskolonne der Energiebedarf für die Feindestillation von Isophorondiamin reduzieren lässt.

Gegenstand der Erfindung ist ein Verfahren zur Feinreinigung von Roh-Isophorondiamin aus der Herstellung von Isophorondiamin durch aminierende Hydrierung von Isophoronnitril in Anwesenheit von mindestens Ammoniak, Wasserstoff, eines Hydrierkatalysator und gegebenenfalls weiteren Zusätzen und in An- oder Abwesenheit von organischen Lösungsmitteln, wobei ein Roh-Isophorondiamin erhalten wird,
dadurch gekennzeichnet, dass das Roh-Isophorondiamin durch zwei Vakuumdestillationskolonnen einer Feinreinigung unterworfen wird, wobei in der ersten Vakuumdestillationskolonne die Abtrennung von noch enthaltenen leichter siedenden Nebenprodukten erfolgt, und in der zweiten Vakuumdestillationskolonne das Isophorondiamin rein über Kopf gewonnen und so von den organischen Rückständen getrennt wird, und wobei die erste Vakuumdestillationskolonne einen aufgesetzten Partialkondensator aufweist.

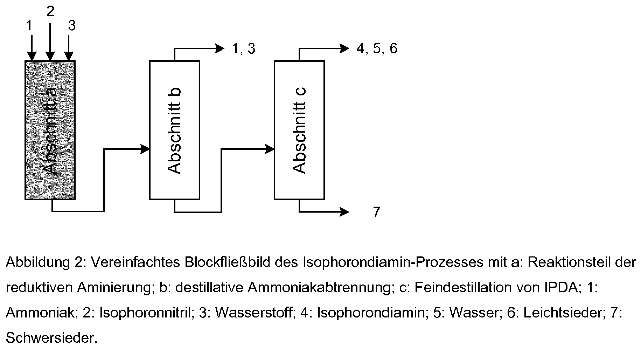

Der gesamte Prozess zur Herstellung von reinem IPDA gliedert sich in drei Abschnitte (siehe Abbildung 2). In Abschnitt a erfolgt die Reaktion durch aminierende Hydrierung von Isophoronnitril in einem ein- oder mehrstufigen Prozess unter Anwesenheit von mindestens Ammoniak, Wasserstoff und einem Katalysator. In Abschnitt b erfolgt die destillative Abtrennung von Ammoniak und Wasserstoff zur Gewinnung von Roh-IPDA. Die Destillation kann in einer oder mehreren Kolonnen durchgeführt werden. In Abschnitt c erfolgt die Feinreinigung vom Roh-IPDA durch destillative Abtrennung von IPDA, Wasser, Leichtsieder und Schwersieder. Die Feinreinigung wird in zwei Kolonnen durchgeführt.

Das Roh-IPDA weißt im Allgemeinen die folgende Zusammensetzung in Gewichts-% (Gew.-%) auf:

| | |
|---|---|
| IPDA | 75-100 Gew.-% |
| Wasser | 0-15 Gew.-% |
| Leichtsieder | 0-6 Gew.-% |
| Schwersieder | 0-6 Gew.-% |

Leichtsieder sind dabei so definiert, dass es sich hierbei um Nebenprodukte aus dem Herstellungsprozess handelt mit einem niedrigeren Siedepunkt als IPDA. Schwersieder sind dabei so definiert, dass es sich hierbei um Nebenprodukte aus dem Herstellungsprozess handelt mit einem höheren Siedepunkt als IPDA.

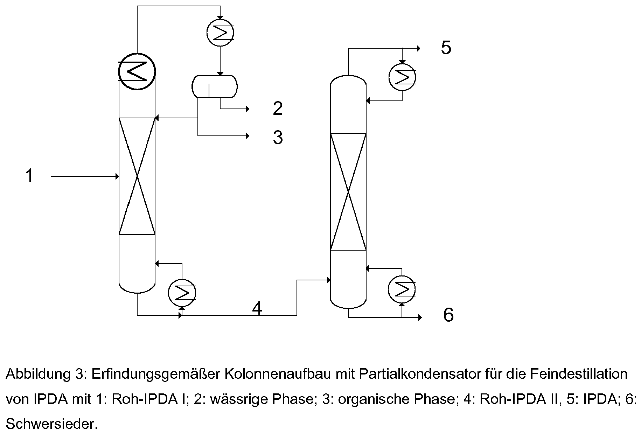

Das Roh-IPDA I wird zunächst in die erste Vakuumdestillationskolonne geleitet, siehe Abbildung 3. Der Partialkondensator wird am Kopf der ersten Vakuumdestillationskolonne installiert und kann sowohl im Inneren der Vakuumdestillationskolonne als auch außerhalb der Vakuumdestillationskolonne angebracht sein. Hierdurch wird der Dampfstrom im Partialkondensator vorgekühlt und somit teilweise kondensiert. Die kondensierte Flüssigkeit wird als Rückstrom in die Vakuumdestillationskolonne verwendet. Der nicht kondensierte Dampfstrom wird anschließend in einem zweiten Kondensator komplett kondensiert und in eine organische Phase 3 und eine wässrige Phase 2 geteilt, wobei die organische Phase 3 teilweise als Rücklauf für die Vakuumdestillationskolonne verwendet wird. Der andere Teil sowie die wässrige Phase dienen als Ausschleusung für Leichtsieder und Wasser. Das IPDA verlässt die Vakuumdestillationskolonne als Sumpfstrom und wird einer zweiten Vakuumdestillationskolonne zur Abtrennung der Schwersieder zugeführt, hier wird das Rein-IPDA am Kopf der zweiten Vakuumdestillationskolonne gewonnen.

Der Partialkondensator wird unter den folgenden Bedingungen betrieben:

| | |
|---|---|
| Temperatur | 40-120°C |
| Druck | 10-200 mbar |

Die eingesetzte 1. Vakuumdestillationskolonne weist folgenden Parameter auf:

| | |
|---|---|
| Druck | 10-200 mbar |
| Sumpftemperatur | 80-200°C |
| Theoretische Stufen | 10-80 |

Die Zusammensetzung des Zustroms (Roh-IPDA II) aus der 1. Vakuumdestillationskolonne in die 2. Vakuumdestillationskolonne weist folgende Zusammensetzung auf:

| | |
|---|---|
| IPDA | 90-100 Gew.-% |
| Schwersieder | 0-10 Gew.-% |

Die eingesetzte 2. Vakuumdestillationskolonne weist folgenden Parameter auf:

| | |
|---|---|
| Druck | 10-200 mbar |
| Kopftemperatur | 80-200°C |
| Theoretische Stufen | 5-50 |

Die Reinheit des Rein-Isophorondiamins soll mindestens 98 Gew.-% betragen.

### Beispiele

### Beispiel 1: Vergleichsbeispiel

Die Simulation der Destillation erfolgte über Aspen Plus. Im Vergleichsbeispiel wurde ein Aufbau, bestehend aus zwei Vakuumdestillationskolonnen gewählt. Am Kopf der ersten Vakuumdestillationskolonne wurde ein Dekanter zur Trennung der beiden flüssigen Phasen verwendet, wobei nur die organische Phase als Rücklauf für die Vakuumdestillationskolonne verwendet wurde. Die erste Vakuumdestillationskolonne wies dabei 36 und die zweite Vakuumdestillationskolonne 15 theoretische Trennstufen auf, bei Rücklauf-zu-Feed-Verhältnissen von 1,1 (erste Vakuumdestillationskolonne) und 1,4 (zweite Vakuumdestillationskolonne). Hierbei wurde der Kolonnendruck der ersten Vakuumdestillationskolonne auf 110 mbar und der der zweiten Vakuumdestillationskolonne auf 80 mbar festgesetzt.

Der eingesetzte Feedstrom hatte folgende Zusammensetzung in Gewichts-% (Gew.-%):

| | |
|---|---|
| IPDA | 88,6 Gew.-% |
| Wasser | 9,1 Gew.-% |
| Leichtsieder | 1,2 Gew.-% |
| Schwersieder | 1,1 Gew.-% |

In der ersten Vakuumdestillationskolonne erfolgte bei einer Sumpftemperatur von 168°C und einer Kopftemperatur von 95°C die Abtrennung der Leichtsieder und des Wassers. Der Feedstrom von der ersten in die zweite Vakuumdestillationskolonne hatte somit folgende Zusammensetzung:

| | |
|---|---|
| IPDA | 98,7 Gew.-% |
| Schwersieder | 1,3 Gew.-% |

In der zweiten Vakuumdestillationskolonne erfolgte bei einer Sumpftemperatur von 187°C und einer Kopftemperatur von 159°C die Feinreinigung von IPDA. Es wurde hierbei eine Reinheit von 99,95 Gew.-% erreicht. Der gesamte IPDA-Verlust über das 2-Vakuumdestillationskolonnensystem betrug 0,2 Gew.-%.

Bei einem gewählten Feedmassenstrom von 2160 kg pro Stunde betrug die erforderliche

| | |
|---|---|
| Heizleistung: | 1184 kW |

| | |
|---|---|
| Kühlleistung: | 1232 kW |

### Beispiel 2: Erfindungsgemäßer Vakuumdestillationskolonnenaufbau

Die Simulation der Destillation erfolgte über Aspen Plus. Im erfindungsgemäßen Beispiel wurde ein Aufbau, bestehend aus zwei Vakuumdestillationskolonnen gewählt. Am Kopf der ersten Vakuumdestillationskolonne wurde ein Dekanter zur Trennung der beiden flüssigen Phasen verwendet. Zusätzlich wurde vor dem Dekanter ein Partialkondensator eingebaut. Der Dampfstrom wurde in dieser Vorrichtung zuerst partiell kondensiert und als Rücklauf in die Vakuumdestillationskolonne gegeben. Der restliche Dampfstrom wurde anschließend totalkondensiert und über den Dekanter in die beiden flüssigen Phasen getrennt. Die organische Phase wurde dabei ebenfalls als Rücklauf für die Vakuumdestillationskolonne verwendet. Die erste Vakuumdestillationskolonne wies 36 und die zweite Vakuumdestillationskolonne 15 theoretische Trennstufen auf, bei Rücklauf-zu-Feed-Verhältnissen von 1,1 (erste Vakuumdestillationskolonne) und 1,4 (zweite Vakuumdestillationskolonne). Hierbei wurde der Kolonnendruck der ersten Vakuumdestillationskolonne auf 110 mbar und der der zweiten Vakuumdestillationskolonne auf 80 mbar festgesetzt.

Der eingesetzte Feedstrom hatte folgende Zusammensetzung in Gewichts-% (Gew.-%):

| | |
|---|---|
| IPDA | 88,6 Gew.-% |
| Wasser | 9,1 Gew.-% |
| Leichtsieder | 1,2 Gew.-% |
| Schwersieder | 1,1 Gew.-% |

In der ersten Vakuumdestillationskolonne erfolgte bei einer Sumpftemperatur von 168°C, einer Kopftemperatur von 117°C und einer Partialkondensatortemperatur von 74°C die Abtrennung der Leichtsieder und des Wassers. Der Feedstrom von der ersten in die zweite Vakuumdestillationskolonne hatte somit folgende Zusammensetzung:

| | |
|---|---|
| IPDA | 98,7 Gew.-% |
| Schwersieder | 1,3 Gew.-% |

In der zweiten Vakuumdestillationskolonne erfolgte bei einer Sumpftemperatur von 187°C und einer Kopftemperatur von 159°C die Feinreinigung von IPDA. Es wurde hierbei eine Reinheit von 99,95 Gew.-% erreicht. Der gesamte IPDA-Verlust über das 2-Kolonnensystem betrug 0,2 Gew.-%.

Bei einem gewählten Feedmassenstrom von 2160 kg pro Stunde betrug die erforderliche

| | |
|---|---|
| Heizleistung: | 1006 kW |
| Kühlleistung: | 1048 kW. |

Die erforderlichen Heiz- und Kühlleistungen beim erfindungsgemäßen Destillationsaufbau mit eingebautem Partialkondensator lagen jeweils um 15% niedriger als beim Vergleichsaufbau.

## Patentansprüche

1. Verfahren zur Feinreinigung von Isophorondiamin aus der Herstellung von Isophorondiamin durch aminierende Hydrierung von Isophoronnitril in Anwesenheit von mindestens Ammoniak, Wasserstoff, eines Hydrierkatalysators und gegebenenfalls weiteren Zusätzen und in An- oder Abwesenheit von organischen Lösungsmitteln, wobei ein Roh-Isophorondiamin erhalten wird,
**dadurch gekennzeichnet, dass** das Roh-Isophorondiamin durch zwei Vakuumdestillationskolonnen einer Feinreinigung unterworfen wird, wobei in der ersten Vakuumdestillationskolonne die Abtrennung von noch enthaltenen leichter siedenden Nebenprodukten erfolgt, und in der zweiten Vakuumdestillationskolonne das Isophorondiamin rein über Kopf gewonnen und so von den organischen Rückständen getrennt wird, und wobei die erste Vakuumdestillationskolonne einen aufgesetzten Partialkondensator aufweist.

2. Verfahren nach Anspruch 1, **dadurch gekennzeichnet, dass** Roh-Isophorondiamin im Allgemeinen die folgende Zusammensetzung in Gewichts-% (Gew.-%) aufweist:
| | |
|---|---|
| IPDA | 75-100 Gew.-% |
| Wasser | 0-15 Gew.-% |
| Leichtsieder | 0-6 Gew.-% |
| Schwersieder | 0-6 Gew.-% |

3. Verfahren nach Anspruch 1-2, **dadurch gekennzeichnet, dass** der Partialkondensator am Kopf der ersten Vakuumdestillationskolonne installiert ist, und im Inneren der Kolonne oder außerhalb der Kolonne angebracht ist.

4. Verfahren nach Anspruch 1-3, **dadurch gekennzeichnet, dass** der Dampfstrom in der ersten Vakuumdestillationskolonne im Partialkondensator vorgekühlt und somit teilweise kondensiert wird und die kondensierte Flüssigkeit als Rückstrom in die Vakuumdestillationskolonne verwendet wird.

5. Verfahren nach Anspruch 1-4, **dadurch gekennzeichnet, dass** der nicht kondensierte Dampfstrom in der ersten Vakuumdestillationskolonne in einem zweiten Kondensator komplett kondensiert und in eine organische Phase und eine wässrige Phase geteilt wird, wobei die organische Phase teilweise als Rücklauf in die erste Vakuumdestillationskolonne verwendet wird.

6. Verfahren nach Anspruch 1-5, **dadurch gekennzeichnet, dass** das IPDA die erste Vakuumdestillationskolonne als Sumpfstrom verlässt und einer zweiten Vakuumdestillationskolonne zur Abtrennung der Schwersieder zugeführt wird.

7. Verfahren nach Anspruch 1-6, **dadurch gekennzeichnet, dass** das Rein-IPDA am Kopf der zweiten Vakuumdestillationskolonne gewonnen wird.

8. Verfahren nach Anspruch 1-7, **dadurch gekennzeichnet, dass** der Partialkondensator unter den folgenden Bedingungen betrieben wird:
| | |
|---|---|
| Temperatur | 40-120°C |
| Druck | 10-200 mbar |

9. Verfahren nach Anspruch 1-8, **dadurch gekennzeichnet, dass** die eingesetzte erste Vakuumdestillationskolonne folgenden Parameter aufweist:
| | |
|---|---|
| Druck | 10-200 mbar |
| Sumpftemperatur | 80-200°C |
| Theoretische Stufen | 10-80 |

10. Verfahren nach Anspruch 1-9, **dadurch gekennzeichnet, dass** die Zusammensetzung des Zustroms aus der ersten Vakuumdestillationskolonne in die zweite Vakuumdestillationskolonne (Roh-IPDA II) folgende Zusammensetzung aufweist:
| | |
|---|---|
| IPDA | 90-100 Gew.-% |
| Schwersieder | 0-10 Gew.-% |

11. Verfahren nach Anspruch 1-10, **dadurch gekennzeichnet, dass** die eingesetzte zweite Vakuumdestillationskolonne folgenden Parameter aufweist:
| | |
|---|---|
| Druck | 10-200 mbar |
| Kopftemperatur | 80-200°C |
| Theoretische Stufen | 5-50 |

## Claims

1. Process for fine purification of isophoronediamine from the production of isophoronediamine by aminating hydrogenation of isophorone nitrile in the presence of at least ammonia, hydrogen, a hydrogenation catalyst and optionally further additions and in the presence or absence of organic solvents to obtain a crude isophoronediamine,
**characterized in that** the crude isophoronediamine is subjected to a fine purification via two vacuum distillation columns, wherein in the first vacuum distillation column the removal of any remaining relatively low-boiling byproducts is effected and in the second vacuum distillation column the isophoronediamine is obtained in pure form as tops and thus separated from the organic residues, and wherein the first vacuum distillation column has a partial condenser fitted to it.

2. Process according to Claim 1, **characterized in that** the crude isophoronediamine generally has the following composition in weight% (wt%):
| | |
|---|---|
| IPDA | 75-100 wt% |
| Water | 0-15 wt% |
| Low boilers | 0-6 wt% |
| High boilers | 0-6 wt% |

3. Process according to Claims 1-2, **characterized in that** the partial condenser is installed at the top of the first vacuum distillation column and is attached inside the column or outside the column.

4. Process according to Claims 1-3, **characterized in that** the vapor stream stream in the first vacuum distillation column is pre-cooled and consequently partly condensed in the partial condenser and the condensed liquid is used as return stream in the vacuum distillation column.

5. Process according to Claims 1-4, **characterized in that** the uncondensed vapor stream in the first vacuum distillation column is fully condensed and divided into an organic phase and an aqueous phase in a second condenser, wherein the organic phase is partly used as reflux for the first vacuum distillation column.

6. Process according to Claims 1-5, **characterized in that** IPDA leaves the first vacuum distillation column as bottoms stream and is sent to a second vacuum distillation column for removal of the high boilers.

7. Process according to Claims 1-6, **characterized in that** the pure IPDA is obtained at the top of the second vacuum distillation column.

8. Process according to Claims 1-7, **characterized in that** the partial condenser is operated under the following conditions:
| | |
|---|---|
| Temperature | 40-120°C |
| Pressure | 10-200 mbar |

9. Process according to Claims 1-8, **characterized in that** the employed first vacuum distillation column has the following parameters:
| | |
|---|---|
| Pressure | 10-200 mbar |
| Bottoms temperature | 80-200°C |
| Theoretical plates | 10-80 |

10. Process according to Claims 1-9, **characterized in that** the composition of the feed stream from the first vacuum distillation column into the second vacuum distillation column (crude IPDA II) has the following composition:
| | |
|---|---|
| IPDA | 90-100 wt% |
| High boilers | 0-10 wt% |

11. Process according to Claims 1-10, **characterized in that** the employed second vacuum distillation column has the following parameters:
| | |
|---|---|
| Pressure | 10-200 mbar |
| Tops temperature | 80-200°C |
| Theoretical plates | 5-50 |

## Revendications

1. Procédé de purification fine d'isophorone-diamine issue de la fabrication d'isophorone-diamine par hydrogénation aminante d'isophorone-nitrile en présence au moins d'ammoniac, d'hydrogène, d'un catalyseur d'hydrogénation et éventuellement d'autres additifs et en présence ou en absence de solvants organiques, une isophorone-diamine brute étant obtenue,
**caractérisé en ce que** l'isophorone-diamine brute est soumise à une purification fine dans deux colonne de distillation sous vide, la séparation de produits secondaires de faible point d'ébullition encore contenus ayant lieu dans la première colonne de distillation sous vide, et l'isophorone-diamine étant obtenue sous forme pure par la tête dans la deuxième colonne de distillation sous vide et ainsi séparée des résidus organiques, la première colonne de distillation sous vide comprenant un condensateur partiel superposé.

2. Procédé selon la revendication 1, **caractérisé en ce que** l'isophorone-diamine brute présente généralement la composition suivante en % en poids :
IPDA : 75 à 100 % en poids
eau : 0 à 15 % en poids
composants de faible point d'ébullition : 0 à 6 % en poids
composants de point d'ébullition élevé : 0 à 6 % en poids.

3. Procédé selon les revendications 1 à 2, **caractérisé en ce que** le condensateur partiel est installé à la tête de la première colonne de distillation sous vide, et est disposé à l'intérieur de la colonne ou à l'extérieur de la colonne.

4. Procédé selon les revendications 1 à 3, **caractérisé en ce que** le courant de vapeur dans la première colonne de distillation sous vide est pré-refroidi dans le condensateur partiel et ainsi partiellement condensé, et le liquide condensé est utilisé en tant que courant de reflux dans la colonne de distillation sous vide.

5. Procédé selon les revendications 1 à 4, **caractérisé en ce que** le courant de vapeur non condensé dans la première colonne de distillation sous vide est complètement condensé dans un deuxième condensateur, et séparé en une phase organique et une phase aqueuse, la phase organique étant utilisée en partie en tant que reflux dans la première colonne de distillation sous vide.

6. Procédé selon les revendications 1 à 5, **caractérisé en ce que** l'IPDA quitte la première colonne de distillation sous vide sous la forme d'un courant de fond et est introduite dans une deuxième colonne de distillation sous vide pour la séparation des composants de point d'ébullition élevé.

7. Procédé selon les revendications 1 à 6, **caractérisé en ce que** l'IPDA pure est obtenue à la tête de la deuxième colonne de distillation sous vide.

8. Procédé selon les revendications 1 à 7, **caractérisé en ce que** le condensateur partiel est exploité dans les conditions suivantes :
| | |
|---|---|
| température : | 40 à 120 °C |
| pression : | 10 à 200 mbar. |

9. Procédé selon les revendications 1 à 8, **caractérisé en ce que** la première colonne de distillation sous vide utilisée présente les paramètres suivants :
| | |
|---|---|
| pression : | 10 à 200 mbar |
| température de fond : | 80 à 200 °C |
| étapes théoriques : | 10 à 80. |

10. Procédé selon les revendications 1 à 9, **caractérisé en ce que** la composition du courant d'alimentation issu de la première colonne de distillation sous vide dans la deuxième colonne de distillation sous vide (IPDA brute II) présente la composition suivante :
| | |
|---|---|
| IPDA : | 90 à 100 % en poids |
| composants de point d'ébullition élevé : | 0 à 10 % en poids. |

11. Procédé selon les revendications 1 à 10, **caractérisé en ce que** la deuxième colonne de distillation sous vide utilisée présente les paramètres suivants :
| | |
|---|---|
| pression : | 10 à 200 mbar |
| température de tête : | 80 à 200 °C |
| étapes théoriques : | 5 à 50. |
